# EUROPÄISCHE PATENTSCHRIFT

(11) **EP 2 613 716 B1**
(45) Veröffentlichungstag und Bekanntmachung des Hinweises auf die Patenterteilung: **12.02.2020**
(21) Anmeldenummer: 11749417.9
(22) Anmeldetag: 30.08.2011
(51) Int. Cl.: A61B 17/122, A61B 17/128, A61B 17/28, A61B 17/30, A61B 90/00, A61B 34/30

(54) **CHIRURGISCHER CLIP**
SURGICAL CLIP
CLIP CHIRURGICAL

(30) Priorität: 10.09.2010 DE 102010037468
(43) Veröffentlichungstag der Anmeldung: 17.07.2013
(73) Patentinhaber: Aesculap AG, 78532 Tuttlingen (DE)
(72) Erfinder: WEIßHAUPT, Dieter, 78194 Immendingen (DE); NESPER, Markus, 78532 Tuttlingen (DE); STEINHILPER, Klaus-Dieter, 78532 Tuttlingen (DE); PLEIL, Thomas, 78073 Bad Dürrheim (DE)
(74) Vertreter: Hoeger, Stellrecht & Partner Patentanwälte mbB
(86) Internationale Anmeldenummer: PCT/EP2011/064923
(87) Internationale Veröffentlichungsnummer: WO 2012/031949

(56) Entgegenhaltungen:
- DE-B3-102006 031 092
- DE-C1- 19 809 121
- US-A- 4 360 023
- US-A- 4 444 187
- US-A- 4 484 581
- US-A- 4 796 625
- US-A- 5 924 176
- US-B1- 6 179 850

## Beschreibung

Die Erfindung betrifft einen chirurgischen Clip, insbesondere zur Verwendung als Implantat, wobei hier die Verwendung als Aneurysmen-Clip wiederum eine hervorragende Bedeutung hat.

Chirurgische Clips mit zwei Clipbranchen und einem federelastischen Element, das die beiden Clipbranchen an einem ihrer Enden miteinander verbindet, sind bekannt.

Die beiden Clipbranchen weisen jeweils ein erstes freies Ende auf, die in Ruhestellung des Clips mit einer vorgegebenen Schließkraft durch das federelastische Element parallel aneinander anliegend gehalten sind und ein zweites, dem freien Ende entgegen gesetztes Ende, das an dem federelastischen Element gehalten ist.

Die Clipbranchen weisen jeweils zwischen ihrem ersten, freien Ende und dem zweiten Ende sich überkreuzend ausgebildete Abschnitte auf. Der Clip kann aus der Ruhestellung in eine Applikationsstellung überführt werden, indem die zweiten Enden mit einem Werkzeug, insbesondere einer Clipanlegezange, gefasst und gegen die Federkraft des federelastischen Elements einander angenähert werden.

Wesentlich bei den chirurgischen Clips ist ihre Schließkraft, die präzise eingestellt sein muss und insbesondere bei der Verwendung des chirurgischen Clips als Implantat auch auf Dauer gegeben sein muss.

Deshalb werden die chirurgischen Clips der Eingangs beschriebenen Art in einem aufwendigen Vorgang auf ihre Schließkraft hin präzise eingestellt und erfordern eine hohe Präzision mit Toleranzen, die teilweise im Hundertstel mm-Bereich liegen. Nur so kann ein reproduzierbares Schließverhalten der Clips erreicht werden.

Um bei der Applikation der Clips das federelastische Element nicht über den elastischen Bereich hinaus zu beanspruchen, werden Clipanlegezangen passend zu den unterschiedlichen Clips bereit gestellt, die so ausgebildet sind, dass eine Überdehnung des Clips und damit eine Verformung des Clips über den elastischen Bereich hinaus nicht vorkommen kann. Dies kann beispielsweise durch eine Begrenzung des Schließwinkels der Zangen geschehen. Für unterschiedliche Clips sind daran angepasste Clipanlegezangen notwendig.

Nachdem für Clips unterschiedlicher Schließkraft unterschiedlich ausgebildete Clipanlegezangen benötigt werden, besteht die Gefahr, dass die Anlegezangen vertauscht werden, mit dem Risiko, dass ein Clip zu weit geöffnet wird und das federelastische Element plastisch verformt wird. Hierdurch würde die für den Clip ausgewiesene Schließkraft vermindert, was für den Patienten, dem ein solches Implantat eingesetzt wurde, eine große Gefahr bedeuten kann.

Aufgabe der vorliegenden Erfindung ist es, einen chirurgischen Clip zu schaffen, der einfach in der Handhabung und gleichwohl sicher als im Implantat einsetzbar ist.

Diese Aufgabe wird erfindungsgemäß durch einen chirurgischen Clip mit den Merkmalen des Anspruchs 1 gelöst. Besondere Ausführungsformen sind in den Unteransprüchen angegeben.

Aufgrund der an den Clipbranchen komplementär zueinander ausgebildeten Anschlagelemente lässt sich unabhängig davon, ob für das Anlegen des Clips eine geeignete Anlegezange verwendet wird oder nicht, garantieren, dass der Clip nicht über seine maximal zulässige Öffnung hinaus geöffnet und das federelastische Element höchstens bis zu seiner maximal zulässigen elastischen Verformung beansprucht wird.

Die Anschlagelemente werden in Flucht mit einer Krafteinwirkungsebene angeordnet, in der bei einer Überführung des Clips aus der Ruhe- in die Applikationsstellung die Kräfte der Clipanlegezange wirken.

Dies vermeidet, dass bei dem aneinander Anliegen der Anschlagelemente Kippmomente entstehen können.

Weiter bevorzugt werden die komplementär ausgebildeten Anschlagelemente, so ausgebildet, dass bei der Überführung in die Applikationsstellung eine Führung der Clipbranchen in lateraler Richtung resultiert. Dies stellt weiterhin sicher, dass die Clipbranchen in der für sie vorgegebenen Ebene in die Applikationsstellung gelangen und in dieser, solange die Kräfte der Clipanlegezange wirken, auch verbleiben und nicht seitlich ausweichen können.

Ein bevorzugtes Beispiel für die komplementäre Ausbildung der Anschlagelemente ist eine Prismenstruktur, bei der die Kanten des Prismas parallel zur Längsrichtung der Clipbranchen ausgerichtet sind. Bevorzugt wird dabei eine Clipbranche mit einer positiven Prismenstruktur ausgebildet, während das andere Anschlagelement die Negativstruktur hierzu aufweist.

Bei einer einfacher gestalteten Variante kann eine negativ ausgebildete Prismenstruktur die ohnehin gegebene, nach außen gewölbte Struktur des zweiten Endes der anderen Clipbranche auf nehmen und ausreichend zentrieren. Auch braucht bei der Prismenstruktur nicht unbedingt eine scharfe Prismenkante den äußersten Bereich bilden. Dieser kann vielmehr auch abgeflacht oder verrundet ausgebildet sein ohne die Funktion des Anschlagelements zu beinträchtigen.

Bei einer weiter bevorzugten Ausführungsform sind die komplementär ausgebildeten Anschlagelemente so ausgebildet, dass bei der Überführung in die Applikationsstellung eine axiale Führung der Clipbranchen resultiert. Dies vermeidet, dass sich eine Verschiebung der Clipbranchen bei Krafteinwirkung, insbesondere von deren freien Enden in der Längsrichtung gesehen, gegeneinander ergibt.

Besonders bevorzugt sind komplementär ausgebildete Anschlagelemente, die sowohl eine laterale als auch eine axiale Führung der Clipbranchen bewirken. Besonders bevorzugte Beispiel für solche komplementär ausgebildeten Anschlagelemente sind Kugel-, Kegel- oder pyramidale Strukturen, wobei diese auch kegelstumpfförmig oder pyramidenstumpfförmig sein können.

Bei einer bevorzugten Ausführungsform der erfindungsgemäßen chirurgischen Clips ist vorgesehen, dass eine der Clipbranchen in dem überkreuzend geformten Bereich mit einer durchgehenden, sich in Längsrichtung der Clipbranche erstreckenden Langöffnung ausgebildet ist, wobei dann die andere Clipbranche mit ihrem überkreuzend geformten Bereich innerhalb der Langöffnung geführt angeordnet wird.

Diese Ausführungsform stellt insbesondere sicher, dass die Ausrichtung der beiden Clipbranchen mit ihrem freien Ende zusätzlich gesichert wird und auch bei erheblicher Krafteinwirkung durch die Clipanlegezange (bei entsprechenden Schließkräften des Clips) nicht unbeabsichtigt eine Ausweichbewegung der Clipbranchen resultieren kann.

Bei Ausführungsformen mit einer Langöffnung lassen sich die Anschlagelemente durch eine Kalibrierung der Langöffnung und/oder des in der Langöffnung geführten Abschnitts der anderen Clipbranche in einfacher Weise realisieren.

Die Langöffnung kann als Durchgangsöffnung innerhalb einer Clipbranche ausgebildet sein. Alternativ kann parallel zu einem Abschnitt der Clipbranche ein Längselement fixiert werden, insbesondere aufgeschweißt werden, welches einen Abstand zur Clipbranche hält, und so die Langöffnung gebildet werden.

Vorzugsweise ist der Abschnitt der Clipbranche, an dem parallel das Längselement fixiert ist, mit einem verminderten Querschnitt, insbesondere einem abgeflachten Querschnitt, ausgebildet, wobei der abgeflachte Teil der Clipbranche Teil der Langöffnung bildet.

Bevorzugt wird das Längselement als ein Flächenelement ausgebildet, dessen eine Oberfläche parallel zu der Abflachung des Abschnitts der Clipbranche angeordnet ist. Optional kann das Flächenelement einen abgewinkelten Abschnitt umfassen, der die Langöffnung, die zwischen dem Längselement und dem Abschnitt der Clipbranche gebildet wird, teilweise überdeckt. Damit lässt sich in einfacher Weise das Längselement mit der Funktion eines Anschlagelements versehen. Insbesondere kann dabei eine Kante des abgewinkelten Bereichs des Flächenelements ein Anschlagelement für die andere Clipbranche bilden.

Bei bevorzugten Ausführungsformen der erfindungsgemäßen chirurgischen Clips werden die Anschlagelemente im Abschnitt der zweiten Enden der Clipbranchen angeordnet.

Bei einer alternativen bevorzugten Ausführungsform können die Anschlagelemente im Abschnitt der sich überkreuzenden Bereiche angeordnet sein, insbesondere bei Ausführungsformen mit einer Langöffnung in deren Bereich.

Das federelastische Element der erfindungsgemäßen chirurgischen Clips kann in unterschiedlicher Weise ausgebildet sein. Gemäß einer bevorzugten Variante der erfindungsgemäßen chirurgischen Clips umfasst das federelastische Element eine halbkreisförmige Federwicklung.

Weiter bevorzugt ist das federelastische Element mit einer eineinhalbfachen Federwicklung ausgestattet.

Zur exakten Positionierung eines Clipanlegewerkzeugs kann vorgesehen sein, dass die erfindungsgemäßen chirurgischen Clips an den außenliegenden Oberflächen der Clipbranchen im Bereich derer zweiten Enden Positionierelemente für das Werkzeug, insbesondere eine Clipanlegezange aufweisen.

Die Positionierelemente können in vielfältiger Form ausgebildet sein, wobei insbesondere Positionierelemente, die als Vertiefung oder als nach außen abstehende Zapfen ausgebildet sind, bevorzugt sind.

Besonders bevorzugt werden die Positionierelemente zu den Anschlagelementen ausgerichtet angeordnet sein, so dass die Positionierelemente, die gleichzeitig auch den Bereich des Kontakts zwischen Clip und Clipanlegezange definieren, die von der Clipanlegezange eingeleitete Kraft im Bereich der Anschlagelemente einleiten und so eine besonders sichere Begrenzung, der auf das federelastische Element einwirkenden Kräfte, erlauben.

Der erfindungsgemäße chirurgische Clip wird, wie eingangs schon erwähnt, insbesondere als Aneurysmen-Clip ausgebildet, insbesondere auch als cerebraler Aneurysmen-Clip.

Diese und weitere Vorteile der Erfindung werden im Folgenden anhand der Zeichnung noch näher erläutert.

Es zeigen im Einzelnen:
- Figur 1: einen erfindungsgemäßem Aneurysmen-Clip von einer Clipanlegezange gehalten;
- Figur 2: den Aneurysmenclip der Figur 1 in vergrößerter Darstellung;
- Figur 3: eine alternative Ausführungsform eines erfindungsgemäßen Clips;
- Figur 4: eine weitere Ausführungsform eines erfindungsgemäßen Clips;
- Figur 5A bis C: eine weitere Ausführungsform eines erfindungsgemäßen Clips in verschiedenen Positionen zu und mit einer Clipanlegezange; und
- Figur 6: eine weitere Ausführungsform eines erfindungsgemäßen Clips.

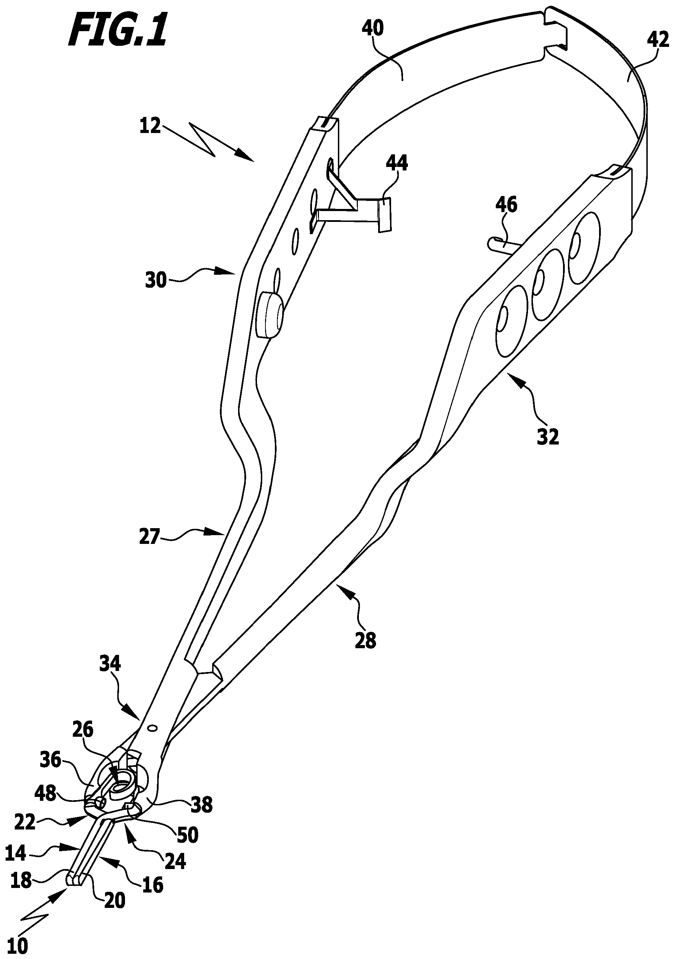

Figur 1 zeigt einen erfindungsgemäßen chirurgischen Clip 10, der in einer Clipanlegezange 12 gehalten ist.

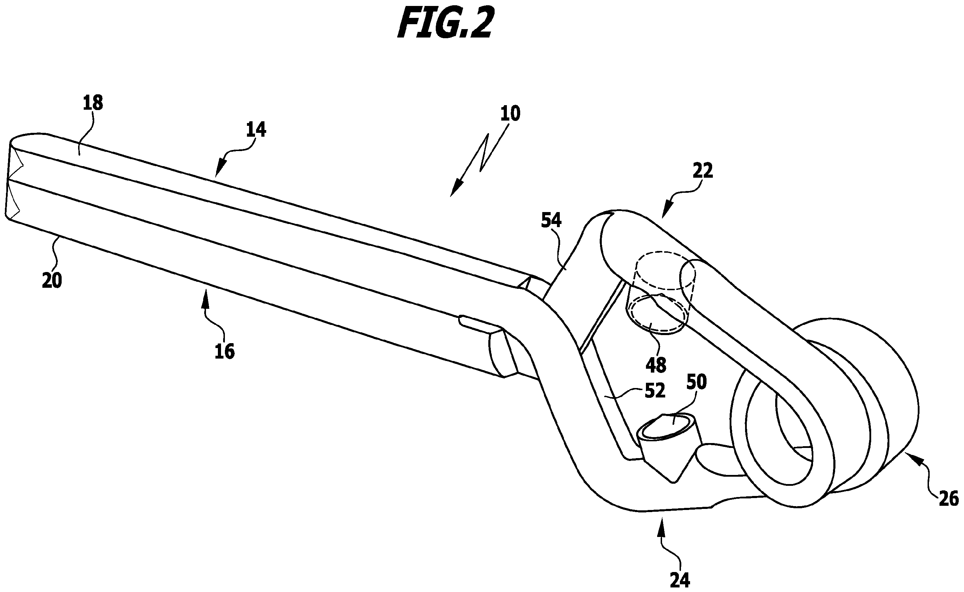

Der erfindungsgemäße chirurgische Clip 10, der im Zusammenhang mit der Figur 2 im Einzelnen noch beschrieben werden wird, umfasst zwei Clipbranchen 14, 16, deren freie erste Enden 18, 20 im geschlossenen Zustand des Clips, d.h. in dessen Ruhestellung, wie in Figur 1 gezeigt, flächig aneinander anliegen.

Die beiden Clipbranchen 14, 16 sind sich überkreuzend ausgebildet und mit ihren den freien ersten Enden 18, 20 entgegen gesetzten zweiten Enden 22, 24 über ein Federelement 26 miteinander verbunden.

Die Clipanlegezange 12 weist zwei Griffbranchen 27, 28 auf, an die sich zwei Griffteile 30, 32 anschließen. Die Griffbranchen 27, 28 sind in ihrem vorderen Bereich 34 gelenkig miteinander verbunden.

Die sich an die Griffbranchen 27, 28 jenseits des Gelenks 34 anschließenden Maulteile 36, 38 der Zange 12 sind mit gegeneinander ausgerichteten konkaven Strukturen ausgebildet, so dass sie die mit dem Federelement 26 verbundenen zweiten Enden 22, 24 der Clipbranchen sicher aufnehmen können.

Am entgegen gesetzten Ende der Griffteile 30, 32 sind zwei Blattfedern 40, 42 angeordnet und mit ihren freien Enden gelenkig verbunden, so dass die Clipanlegezange im unbetätigten Zustand keinen Druck auf den Clip 10 ausübt und sich die Clipanlegezange kraftlos von dem Clip 10 lösen lässt.

Zum Anlegen, des erfindungsgemäßen chirurgischen Clips 10 werden die Griffteile 30, 32 gegeneinander gedrückt und bei Bedarf in diesem Zustand über Verriegelungselemente 44, 46 gehalten. In diesem Zustand drücken die Maulteile 36, 38 die zweiten Enden 22, 24 der Clipbranchen 14, 16 gegeneinander und öffnen damit den Clip mit seinen freien Enden 18, 20 in seine Applikationsstellung.

Der maximale Öffnungswinkel der freien Enden 18, 20, d.h. die Applikationsstellung, wird bestimmt durch Anlageelemente 48, 50. In dieser Weise wird der Öffnungswinkel des erfindungsgemäßen Clips 10 begrenzt, so dass sichergestellt ist, dass das Federelement 26 lediglich innerhalb des elastischen Bereiches verformt wird und die für den Clip ausgewiesene Schließkraft nach der Applikation erhalten bleibt.

Figur 2 zeigt den erfindungsgemäßen chirurgischen Clip 10 aus Figur 1 im Detail in der Ruhestellung, d.h. die beiden freien ersten Enden 18, 20 der beiden Clipbranchen 14, 16 liegen in paralleler Anordnung aneinander an.

Die beiden Clipbranchen 14 und 16 sind über das federelastische Element 26 miteinander verbunden, welches eine eineinhalbfache Federwicklung umfasst.

Die zweiten Enden 22, 24 der beiden Clipbranchen 14, 16 umfassen jeweils ein nach innen gerichtetes Anlageelement 48, 50 auf, wobei das Anlageelement 48 eine kegelförmige Vertiefung aufweist und das Anlageelement 50 eine korrespondierend ausgeformte kegelförmige Erhebung.

Werden die beiden zweiten Enden 22, 24 der beiden Clipbranchen 16, 14 gegeneinander gedrückt, rücken im maximal geöffneten Zustand (Applikationsstellung) der freien Enden 18, 20 die kegelförmige Erhebung des Anlageelements 50 in die kegelförmige Ausnehmung des Anlageelements 48 ein und Verhindern eine weitere Öffnung des chirurgischen Clips 10.

Damit ist sicher gestellt, dass das federelastische Element 26 nur innerhalb seines elastischen Bereichs verformt wird, somit der plastische Bereich vermieden und die Schließkraft, die zuvor bei der Herstellung exakt eingestellt wurde, für den Clip erhalten bleibt.

Die Bereiche der Clipbranchen 14, 16 zwischen den freien Enden 18, 20 und den zweiten Enden 22, 24 sind überkreuzend ausgebildet, derart, dass die Branche 18 eine sich in Längsrichtung der Branche erstreckende Öffnung 52 aufweist, durch die ein korrespondierender, abgeflachter Abschnitt 54 der Clipbranche 16 hindurchgeführt ist. Durch die Führung der Clipbranche 16 im Bereich ihres Abschnitts 54 in der Längsöffnung 52 der Clipbranche 14 wird bereits gewährleistet, dass die Ausrichtung der beiden freien Enden 18 und 20 zueinander im Wesentlichen während des gesamten Bewegungsablaufs, während dem die freien Enden 18, 20 aus der Ruhestellung, wie in Figur 2 gezeigt, in eine Applikationsstellung überführt werden, erhalten bleibt.

Die Anlageelemente 48, 50 sorgen zum Einen dafür, dass die Öffnung des Clips nicht über die maximal zulässige Öffnungsstellung (Applikationsstellung) hinaus fortgesetzt wird, und zum Anderen wird über den kegelförmigen Vorsprung des Anlageelement 50 in Zusammenwirkung mit dem kegelartigen Rücksprung des Anlageelement 48 zusätzlich noch eine exakte Zentrierung der zweiten Enden 22, 24 und damit eine exakte Ausrichtung der freien Enden 18, 20 in der Applikationsstellung erzielt, die sowohl in lateraler als auch in Längsrichtung wirkt.

Dieses Ergebnis wird unabhängig davon erhalten, ob nun speziell geeignete Clip-Applikationszangen für den Clip 10 verwendet werden oder nicht.

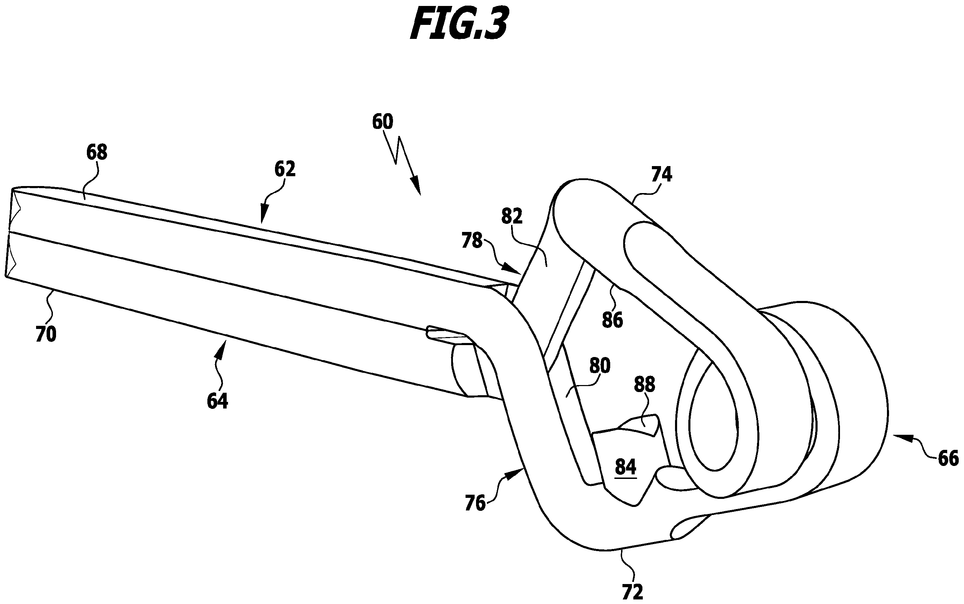

Figur 3 zeigt eine weitere Ausführungsform eines erfindungsgemäßen chirurgischen Clips 60 mit zwei Clipbranchen 62, 64, die über ein federelastisches Element 66 miteinander verbunden sind. Das federelastische Element 66 wurde während der Produktion des Clips 60 wiederum exakt auf eine vorgegebene Schließkraft des Clips 60 eingestellt.

In der in Figur 3 gezeigten geschlossenen Stellung (Ruhestellung) des Clips 60 liegen zwei freie erste Enden 68, 70 der Clipbranchen 62, 64 flächig aneinander.

Die zweiten, den freien Enden 68, 70 entgegen gesetzten Enden 72, 74 der beiden Clipbranchen 62, 64 sind mit dem Federelement 66 verbunden. Die zwischen den jeweiligen Enden 68, 72 bzw. 70, 74 liegenden mittleren Bereiche 76, 78 der beiden Clipbranchen 62 bzw. 64 sind einander überkreuzend ausgestaltet, wobei der mittlere Bereich 76 der einen Clipbranche 62 eine Langöffnung oder ein Langloch 80 aufweist in dem ein abgeflachter Teil 82 des mittleren Bereichs 78 der anderen Clipbranche 64 geführt ist.

An den beiden Enden 72, 74 weist der Clip 60 Anlageelemente 84, 86 auf, von denen das Anlageelement 84 sich zapfenförmig in Richtung zum zweiten Ende 74 der Branche 64 hin erstreckt.

Das Anlageelement 86 der Branche 64 wird durch die Oberfläche des im Wesentlichen zylindrisch ausgeführten zweiten Endes 74 der Branche 64 gebildet.

Zur Aufnahme und Zentrierung in lateraler Richtung weist das Anlageelement 84 eine prismen- oder keilförmige Vertiefung 88 auf in die die zylindrische Oberfläche 86 des zweiten Endes 74 der Clipbranche 64 einrückt und dabei zentriert wird.

Das federelastische Element 66 des Clips 60 ist mit einer anderthalbfachen Federwicklung ausgestattet.

Alternativ zu der Oberfläche 86 des zylindrischen Endes 74 könnte auch vorgesehen sein, dass korrespondierend zu dem Anlageelement 84 ein zapfenförmiger Vorsprung, der in Richtung zum zweiten Ende 72 der Clipbranche 62 weist, anstelle der Oberfläche 86 vorgesehen wird, der dann vorzugsweise mit einem prismenförmigen vorspringenden Querschnitt ausgestattet ist, der komplementär mit dem Rücksprung 88 ausgebildet ist und eine noch präzisere laterale Ausrichtung der beiden Enden 72, 74 in der Applikationsstellung zueinander erlaubt.

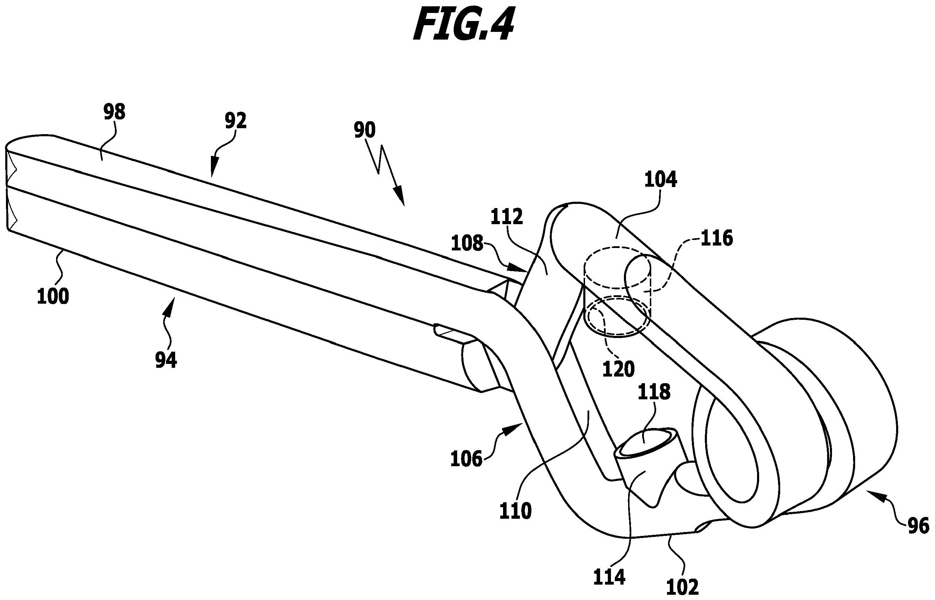

Figur 4 zeigt einen erfindungsgemäßen chirurgischen Clip 90 wiederum in einer geschlossenen Stellung (Ruhestellung), bei der die zwei freien ersten Enden 98, 100 der Clipbranchen 92, 94 aneinander liegen.

Die zweiten, den freien Enden 98, 100 entgegen gesetzten Enden 102, 104 der beiden Clipbranchen 92, 94 sind mit einem Federelement 96 verbunden. Die zwischen den jeweiligen Enden 98, 100 bzw. 102, 104 liegenden mittleren Bereiche 106, 108 der beiden Clipbranchen 92 bzw. 94 sind einander überkreuzend ausgestaltet, wobei der mittlere Bereich 106 der Clipbranche 92 eine Langöffnung oder ein Langloch 110 aufweist in dem ein abgeflachter Teil 112 des mittleren Bereichs 108 der Clipbranche 94 geführt ist.

An den beiden Enden 102, 104 weist der Clip 90 Anlageelemente 114, 116 auf, von denen das Anlageelement 114 sich zapfenförmig in Richtung zum zweiten Ende 104 der Branche 94 hin erstreckt und mit einem kugeligen Abschnitt 118 endet.

Das Anlageelement 116 der Branche 94 wird ebenfalls von einem zapfenförmigen Vorsprung gebildet, an dessen dem Anlageelement 114 zugewandten Ende eine kugelschalenförmige Ausnehmung 120 ausgebildet ist.

Das federelastische Element 96 des Clips 90 ist mit einer anderthalbfachen Federwicklung ausgestattet.

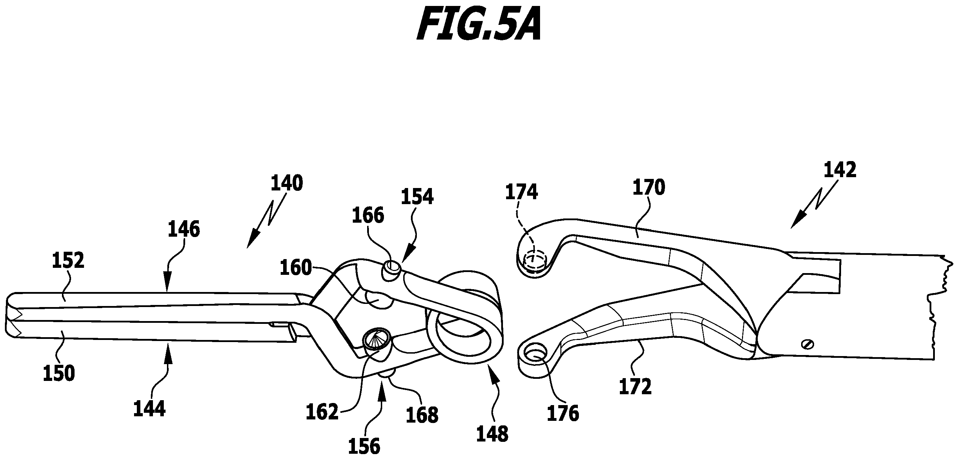

In den Figuren 5A bis 5C ist nochmals anhand einer weiteren Ausführungsform eines erfindungsgemäßen chirurgischen Clips 140 die Handhabung des Clips im Zusammenhang mit einer nur teilweise dargestellten Clipanlegezange 142 schematisch gezeigt.

Der Clip 140 weist zwei Clipbranchen 144, 146 auf, deren freie erste Enden 150, 152 in der Ruhestellung parallel aneinander liegen und deren zweite Enden 154, 156 über ein federelastisches Element 148 miteinander verbunden sind.

Die Ausgestaltung des erfindungsgemäßen Clips 140 entspricht im Wesentlichen dem Clip 10, der in Figur 2 dargestellt ist, so dass auf die Ausführungen dort im Wesentlichen verwiesen werden kann.

Im Bereich zwischen den Enden 150, 154 bzw. 152, 156 sind sich überkreuzende mittlere Bereiche der beiden Clipbranchen 144,146 angeordnet, die in gleicher Weise ausgestaltet sind, wie dies in Figur 2 anhand des Clip 10 gezeigt und erläutert wurde.

Auch weist der Clip 140 Anlageelemente 160, 162 auf, welche einen kegelförmigen Rücksprung (im Anlageelement 160; nicht dargestellt) und einen kegelförmigen Vorsprung am Anlageelement 162 beinhaltet.

Zusätzlich zu den Charakteristika des chirurgischen Clips 10 der Figur 2 weist nun der chirurgische Clip 140 der Figuren 5A bis 5C an den zweiten Enden 154, 156 nach außen abstehende zylindrische Vorsprünge 166, 168 auf, die bei einer Handhabung des Clips 140 der sicheren Verbindung mit der Clipanlegezange 142 und insbesondere einer exakten Platzierung des Clips 140 zwischen Maulteilen 170, 172 der Clipanlagezange 142 dienen. Zu diesem Zweck weisen die beiden Maulteile 170, 172 an ihren freien Enden Vertiefungen 174, 176 auf, die komplementär zu den zapfenförmigen Vorsprüngen 166, 168 des Clips 140 ausgebildet sind und diese zentrierend aufnehmen.

Aus der Figur 5A ist ersichtlich, dass die Maulteile 170, 172 der Clipanlegezange 142 abgekröpft ausgebildet sein können um so den Clip 140 in einer seitlichen Lage zu erfassen.

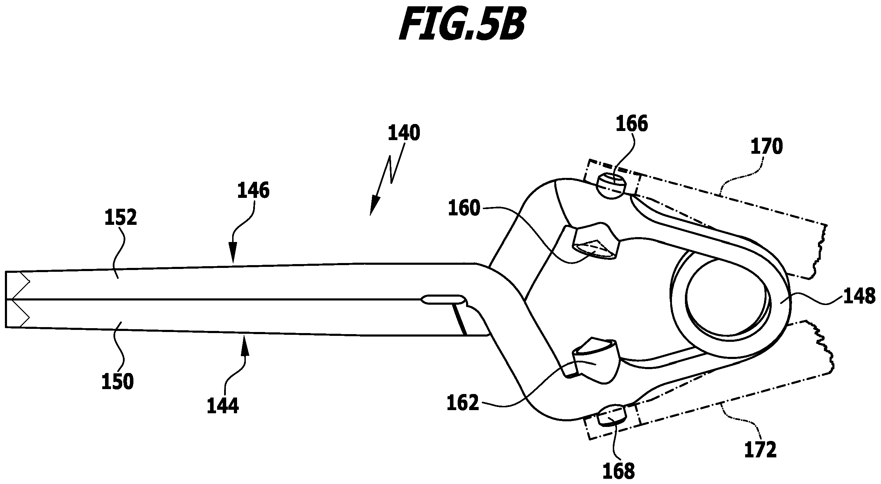

In Figur 5B ist die Situation des Clips 140 in Ruhestellung dargestellt, bei der die beiden Maulteile 170, 172 bereits über die beiden Zapfen 166, 168 zent-riert und mit den in den Maulteilenden vorgesehenen Öffnungen 174, 176 angedockt sind.

Die beiden freien Enden 150, 152 der Clipbranchen 144, 146 sind Figur 5B immer noch in der Ruhestellung gezeigt.

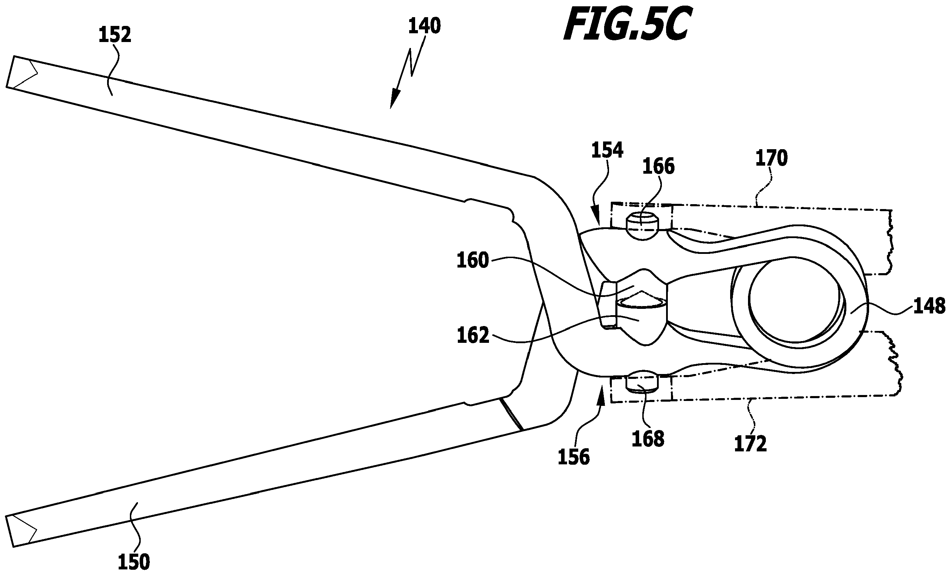

Figur 5C zeigt die Situation, wenn über die Clipanlegezange 142 bzw. über deren Maulteile 170, 172 die Enden 154, 156 zusammengepresst werden bis die beiden Anlageelemente 160, 162 in Eingriff und in Anlage zueinander kommen und so der Clip 140 in die Applikationsstellung überführt ist. Die Maulteile 170, 172 der Clipanlegezange 142 bleiben über die zapfenförmigen Vorsprünge 166, 168 zentriert und fixiert an der korrekten Position des Clips 140 und, wie aus Figur 5C ersichtlich ist, die Krafteinleitung über die Bereiche benachbart zu den zapfenförmigen Vorsprüngen 166, 168 wird im Wesentlichen linear über die Anlageelemente 160, 162 aufgefangen.

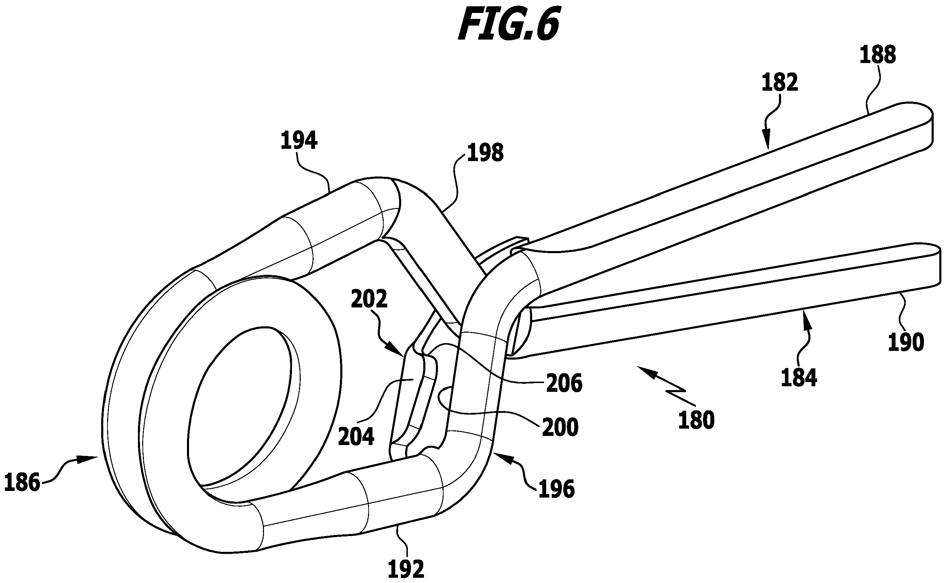

Figur 6 zeigt eine weitere Ausführungsform eines erfindungsgemäßen chirurgischen Clips 180, der in einer Stellung zwischen der Ruhestellung und der Applikationsstellung, d.h. partiell geöffnet, dargestellt ist.

Der erfindungsgemäße Clip 180 weist zwei Clipbranchen 182, 184 auf, die über ein federelastisches Element 186, welches eine eineinhalbfache Federwicklung umfasst, mit einander verbunden sind. Das federelastische Element 186 wurde bei der Produktion des Clips exakt auf eine vorgegebene Schließkraft des Clips eingestellt.

Die zwei ersten freien Enden 188, 190 der Clipbranchen 182, 184 sind von einander leicht beabstandet gezeigt, entsprechend der leicht geöffneten Stellung des Clips 180.

Die zweiten, den freien Enden 188, 190 entgegen gesetzten Enden 192, 194 der beiden Clipbranchen 182, 184 sind mit dem Federelement 186 direkt verbunden. Die zwischen den jeweiligen Enden 188, 192 bzw. 190, 194 liegenden Abschnitte 196, 198 der beiden Clipbranchen 182, 184 sind einander überkreuzend ausgestaltet, wobei der mittlere Abschnitt 196 der einen Clipbranche 182 einen rückspringenden Bereich 200 aufweist, an dem ein abgeflachter Teil des mittleren Abschnitts 198 der anderen Clipbranche 184 geführt ist.

Der rückspringende Bereich 200 des mittleren Abschnitts 196 der Clipbranche 182 wird von einem Längselement 202 überbrückt, das jeweils an seinen beiden Enden mit dem mittleren Abschnitt 196 der Clipbranche 182, beispielsweise durch verschweißen, verbunden ist. Damit bildet das Längselement 202 zusammen mit dem rückspringenden Bereich 200 des mittleren Abschnitts 196 der Clipbranche 182 eine Langöffnung in der dann der abgeflachte Teil des mittleren Abschnitts 198 der Clipbranche 184 geführt ist.

Bevorzugt ist das Längselement 202 aus Flachmaterial gebildet, wobei weiter bevorzugt das Längselement 202 einen in Richtung der Langöffnung gerichteten Vorsprung 204 aufweist, der hier in einfacher Weise durch ein Abwinkeln des Flachmaterials des Längselements 202 gebildet ist.

Die in der Figur 6 nach oben weisende Kante 206 des Längselements 202 dient gleichzeitig als Anschlagelement, das mit der komplementären Unterseite des mittleren Abschnitts 198 der Clipbranche 184 als weiterem Anschlagelement zusammen die maximale Öffnung des Clips 180 und damit die maximale elastische Verformung des Federelements 186 begrenzt.

## Patentansprüche

1. Chirurgischer Clip (10), insbesondere zur Verwendung als Implantat, mit zwei Clipbranchen (14, 16) und einem federelastischen Element (26), über das die beiden Clipbranchen (14, 16) miteinander verbunden sind, wobei die beiden Clipbranchen (14, 16) jeweils ein erstes freies Ende aufweisen, welche in einer Ruhestellung des Clips (10) mit einer vorgegebenen Schließkraft durch das federelastische Element (26) parallel aneinander anliegend gehalten sind, wobei die Clipbranchen (14, 16) an ihren dem freien Ende entgegen gesetzten zweiten Ende an dem federelastischen Element (26) gehalten sind, und wobei die Clipbranchen (14, 16) zwischen ihren ersten und zweiten Enden sich überkreuzend ausgebildete Abschnitte aufweisen, **dadurch gekennzeichnet, dass** die Clipbranchen (14, 16) komplementär zueinander ausgebildete Anschlagelemente (48, 50) aufweisen, welche beim Überführen des Clips (10) aus der Ruhestellung in eine Applikationsstellung, bei der die freien Enden der Clipbranchen (14, 16) voneinander beabstandet sind, gegenseitig in Anlage bringbar sind, wobei die komplementär ausgebildeten Anschlagelemente (48, 50) in der Flucht mit einer Krafteinwirkungsebene angeordnet sind, in der die Überführung des Clips (10) aus der Ruhein die Applikationsstellung vorgenommen wird.

2. Chirurgischer Clip nach Anspruch 1, **dadurch gekennzeichnet, dass** die komplementär ausgebildeten Anschlagelemente (48, 50) so ausgebildet sind, dass bei der Überführung in die Applikationsstellung eine laterale Führung der Clipbranchen (14, 16) resultiert.

3. Chirurgischer Clip nach Anspruch 2, **dadurch gekennzeichnet, dass** die komplementär ausgebildeten Anschlagelemente (48, 50) eine Keil- oder Prismenstruktur umfassen.

4. Chirurgischer Clip nach Anspruch 1 oder 2, **dadurch gekennzeichnet, dass** die komplementär ausgebildeten Anschlagelemente (48, 50) so ausgebildet sind, dass bei der Überführung in die Applikationsstellung eine axiale Führung der Clipbranchen (14, 16) resultiert.

5. Chirurgischer Clip nach einem der Ansprüche 1, 2 oder 4, **dadurch gekennzeichnet, dass** die komplementär ausgebildeten Anschlagelemente (48, 50) so ausgebildet sind, dass bei der Überführung der Clipbranchen (14, 16) aus der Ruhe- in die Applikationsstellung eine laterale und axiale Führung resultiert, wobei vorzugsweise die komplementär ausgebildeten Anschlagelemente (48, 50) eine Kugel-, Kegel- oder pyramidale Struktur umfassen.

6. Chirurgischer Clip nach Anspruch 1 bis 5, **dadurch gekennzeichnet, dass** eine der Clipbranchen (14, 16) in ihrem überkreuzenden Bereich mit einer durchgehenden, sich in Längsrichtung der Clipbranche ersteckenden Langöffnung (52) ausgebildet ist und dass die andere Clipbranche mit ihrem überkreuzenden Bereich innerhalb der Langöffnung (52) geführt angeordnet ist, wobei optional die Anschlagelemente (14, 16) in der Langöffnung (52) der einen und an dem innerhalb der Langöffnung (52) geführten Abschnitt der anderen Clipbranche angeordnet sind.

7. Chirurgischer Clip nach einem der Ansprüche 1 bis 6, **dadurch gekennzeichnet, dass** die Anschlagelemente (48, 50) im Abschnitt der zweiten Enden der Clipbranchen (14, 16) angeordnet sind.

8. Chirurgischer Clip nach einem der Ansprüche 1 bis 6, **dadurch gekennzeichnet, dass** die Anschlagelemente (48, 50) im Abschnitt der sich überkreuzenden Bereiche angeordnet sind.

9. Chirurgischer Clip nach einem der Ansprüche 6 bis 8, **dadurch gekennzeichnet, dass** die Langöffnung (52) in einem Abschnitt einer der Clipbranchen, der vorzugsweise einen Bereich mit vermindertem Querschnitt aufweist, zusammen mit einem parallel daran fixierten Längselement gebildet ist.

10. Chirurgischer Clip nach einem der Ansprüche 1 bis 9, **dadurch gekennzeichnet, dass** das federelastische Element (26) eine Halbkreis-förmige Federwicklung oder eine eineinhalbfache Federwicklung umfasst.

11. Chirurgischer Clip nach einem der Ansprüche 1 bis 10, **dadurch gekennzeichnet, dass** die Clipbranchen (14, 16) an ihren außen liegenden Oberflächen im Bereich ihrer zweiten Enden Positionierelemente für ein Werkzeug aufweisen.

12. Chirurgischer Clip nach Anspruch 11, **dadurch gekennzeichnet, dass** die Positionierelemente zu den Anschlagelementen ausgerichtet angeordnet sind.

13. Chirurgischer Clip nach Anspruch 11 oder 12, **dadurch gekennzeichnet, dass** die Positionierelemente als Vertiefung oder nach außen abstehende Zapfen ausgebildet sind.

14. Chirurgischer Clip nach einem der Ansprüche 1 bis 13, **dadurch gekennzeichnet, dass** der Clip (10) als Aneurysmen-Clip, insbesondere als cerebraler Aneurysmen-Clip ausgebildet ist.

## Claims

1. Surgical clip (10), in particular for use as an implant, comprising two clip arms (14, 16) and a resiliently flexible element (26) via which the two clip arms (14, 16) are connected to each other, wherein the two clip arms (14, 16) each have a first free end, these first free ends being held parallel to and in contact with each other with a predetermined closing force by the resiliently flexible element (26) when the clip (10) is in a rest position, wherein the clip arms (14, 16) are held at a second end thereof opposite the free end on the resiliently flexible element (26) and wherein the clip arms (14, 16) have between their first end and second end sections configured to cross each other, **characterized in that** the clip arms (14, 16) have stop elements (48, 50) configured to complement each other, said stop elements being adapted to be brought in mutual contact when the clip (10) is transferred from the rest position to an application position in which the free ends of the clip arms (14, 16) are spaced apart from each other, wherein the complementary-configured stop elements (48, 50) are arranged in line with a force application plane in which the transfer of the clip (10) from the rest position to the application position is carried out.

2. Surgical clip in accordance with claim 1, **characterized in that** the complementary-configured stop elements (48, 50) are configured such that lateral guiding of the clip arms (14, 16) results when transferred to the application position.

3. Surgical clip in accordance with claim 2, **characterized in that** the complementary-configured stop elements (48, 50) comprise a wedge structure or a prism structure.

4. Surgical clip in accordance with claim 1 or 2, **characterized in that** the complementary-configured stop elements (48, 50) are configured such that axial guiding of the clip arms (14, 16) results when transferred to the application position.

5. Surgical clip in accordance with any one of claims 1, 2 or 4, **characterized in that** the complementary-configured stop elements (48, 50) are configured such that lateral and axial guiding results when the clip arms (14, 16) are transferred from the rest position to the application position, wherein the complementary-configured stop elements (48, 50) preferably comprise a ball structure, a cone structure or a pyramidal structure.

6. Surgical clip in accordance with claims 1 to 5, **characterized in that** one of the clip arms (14, 16) is formed in the crossing region thereof with an elongated opening (52) therethrough extending in a longitudinal direction of the clip arm and **in that** the other clip arm is arranged to be guided with the crossing region thereof within the elongated opening (52), optionally wherein the stop elements (14, 16) are arranged in the elongated opening (52) of the one clip arm and on the section of the other clip arm that is guided within the elongated opening (52).

7. Surgical clip in accordance with any one of claims 1 to 6, **characterized in that** the stop elements (48, 50) are arranged in the section of the second ends of the clip arms (14, 16).

8. Surgical clip in accordance with any one of claims 1 to 6, **characterized in that** the stop elements (48, 50) are arranged in the section of the crossing regions.

9. Surgical clip in accordance with any one of claims 6 to 8, **characterized in that** the elongated opening (52) is formed in a section of one of the clip arms which preferably has an area of reduced cross-section, together with a longitudinal element fixed thereto in parallel.

10. Surgical clip in accordance with any one of claims 1 to 9, **characterized in that** the resiliently flexible element (26) comprises a semi-circular spring coil or a spring coil of one-and-a-half turns.

11. Surgical clip in accordance with any one of claims 1 to 10, **characterized in that** the clip arms (14, 16) have at their exterior surfaces in the area of the second ends thereof positioning elements for a tool.

12. Surgical clip in accordance with claim 11, **characterized in that** the positioning elements are arranged in alignment with the stop elements.

13. Surgical clip in accordance with claim 11 or 12, **characterized in that** the positioning elements are configured as a depression or outward-protruding pins.

14. Surgical clip in accordance with any one of claims 1 to 13, **characterized in that** the clip (10) is configured as an aneurysm clip, in particular as a cerebral aneurysm clip.

## Revendications

1. Clip chirurgical (10), en particulier destiné à être utilisé comme implant, avec deux branches de clip (14, 16) et un élément à élasticité de ressort (26) par le biais duquel les deux branches de clip (14, 16) sont liées l'une à l'autre, où les deux branches de clip (14, 16) présentent chacune une première extrémité libre, qui dans une position de repos du clip (10) sont maintenues avec une force de fermeture prédéterminée en appui l'une contre l'autre parallèlement par l'élément à élasticité de ressort (26), où les branches de clip (14, 16) sont maintenues au niveau de leurs secondes extrémités opposées à l'extrémité libre sur l'élément à élasticité de ressort (26), et où les branches de clip (14, 16) présentent des sections agencées en se croisant entre leurs premières et secondes extrémités, **caractérisé en ce que** les branches de clip (14, 16) présentent des éléments de butée (48, 50) agencés de manière complémentaire l'un à l'autre, qui lors du passage du clip (10) de la position de repos dans une position d'application dans laquelle les extrémités libres des branches de clip (14, 16) sont distantes l'une de l'autre, peuvent être amenés mutuellement en butée, où les éléments de butée (48, 50) agencés de manière complémentaire sont disposés en alignement avec un plan d'application de force dans lequel le passage du clip (10) de la position de repos dans la position d'application est réalisé.

2. Clip chirurgical selon la revendication 1, **caractérisé en ce que** les éléments de butée (48, 50) agencés de manière complémentaire sont agencés de telle manière que, lors du passage dans la position d'application, il en résulte un guidage latéral des branches de clip (14, 16).

3. Clip chirurgical selon la revendication 2, **caractérisé en ce que** les éléments de butée (48, 50) agencés de manière complémentaire comprennent un structure de coin ou de prisme.

4. Clip chirurgical selon la revendication 1 ou 2, **caractérisé en ce que** les éléments de butée (48, 50) agencés de manière complémentaire sont agencés de telle manière que lors du passage dans la position d'application, il en résulte un guidage axial des branches de clip (14, 16).

5. Clip chirurgical selon l'une des revendications 1, 2 ou 4, **caractérisé en ce que** les éléments de butée (48, 50) agencés de manière complémentaire sont agencés de telle manière que lors du passage des branches de clip (14, 16) de la position de repos dans la position d'application il en résulte un guidage latéral et axial, où de préférence les éléments de butée (48, 50) agencés de manière complémentaire comprennent une structure sphérique, conique ou pyramidale.

6. Clip chirurgical selon les revendications 1 à 5, **caractérisé en ce que** l'une des branches de clip (14, 16) est agencée dans son domaine de croisement avec une ouverture longitudinale (52) continue qui s'étend dans la direction longitudinale des branches de clip et **en ce que** l'autre branche de clip est agencée guidée à l'intérieur de l'ouverture longitudinale (52) avec son domaine de croisement, où éventuellement les éléments de butée (14, 16) sont disposés dans l'ouverture longitudinale (52) de l'une des branches de clip et au niveau de la section guidée à l'intérieur de l'ouverture longitudinale (52) de l'autre branche de clip.

7. Clip chirurgical selon l'une des revendications 1 à 6, **caractérisé en ce que** les éléments de butée (48, 50) sont disposés dans la section des secondes extrémités des branches de clip (14, 16).

8. Clip chirurgical selon l'une des revendications 1 à 6, **caractérisé en ce que** les éléments de butée (48, 50) sont disposés dans la section des domaines qui se croisent.

9. Clip chirurgical selon l'une des revendications 6 à 8, **caractérisé en ce que** l'ouverture longitudinale (52) est formée dans une section de l'une des branches de clip qui présente de préférence un domaine à section droite réduite, en même temps qu'un élément longitudinal fixé à celle-ci parallèlement.

10. Clip chirurgical selon l'une des revendications 1 à 9, **caractérisé en ce que** l'élément à élasticité de ressort (26) comprend un enroulement de ressort en forme de demi-cercle ou un enroulement de ressort d'une fois et demie.

11. Clip chirurgical selon l'une des revendications 1 à 10, **caractérisé en ce que** les branches de clip (14, 16) présentent au niveau de leurs surfaces situées à l'extérieur dans le domaine de leurs secondes extrémités des éléments de positionnement pour un outil.

12. Clip chirurgical selon la revendication 11, **caractérisé en ce que** les éléments de positionnement sont disposés en étant dirigés vers les éléments de butée.

13. Clip chirurgical selon la revendication 11 ou 12, **caractérisé en ce que** les éléments de positionnement sont agencés comme un évidement ou des tenons en saillie vers l'extérieur.

14. Clip chirurgical selon l'une des revendications 1 à 13, **caractérisé en ce que** le clip (10) est agencé comme un clip d'anévrisme, en particulier comme un clip d'anévrisme cérébral.
